Europäisches Patentamt

European Patent Office

Office européen des brevets

(19)

(11) EP 0 793 955 B1

(12) **FASCICULE DE BREVET EUROPEEN**

(45) Date de publication et mention
de la délivrance du brevet:
**20.12.2000 Bulletin 2000/51**

(51) Int Cl.$^7$: **A61K 7/00**

(21) Numéro de dépôt: **97400299.0**

(22) Date de dépôt: **10.02.1997**

(54) **Dispositifs pressurisés comprenant une èmulsion huile dans eau moussante ultrafine**

Unter Druck stehende Spender mit ultrafeiner schäumender O/W-Emulsion

Pressurised dispensers comprising an ultrafine foaming o/w emulsion

(84) Etats contractants désignés:
**DE ES FR GB IT**

(30) Priorité: **07.03.1996 FR 9602898**

(43) Date de publication de la demande:
**10.09.1997 Bulletin 1997/37**

(73) Titulaire: **L'OREAL**
**75008 Paris (FR)**

(72) Inventeur: **Lorant, Raluca**
**94320 Thiais (FR)**

(74) Mandataire: **Miszputen, Laurent**
**L'Oreal,**
**DPI,**
**6 rue Sincholle**
**92585 Clichy Cédex (FR)**

(56) Documents cités:
EP-A- 0 641 557          WO-A-92/16188
WO-A-95/05796           WO-A-95/17163
WO-A-96/02225           DE-A- 4 243 272
DE-A- 4 318 171          FR-A- 2 697 160
US-A- 5 145 604

- DATABASE WPI Week 9137 Derwent
  Publications Ltd., London, GB; AN 91-270690
  XP002020516 "Microemulsion of high stability,
  useful for cosmetics and medicine - contg. liq.
  oily substances, surfactants vitamin-E and
  water, mixed at hign pressure to form emulsion
  of specific mean dia." & JP 03 178 331 A
  (KANEBO) , 2 août 1991
- Kosmetik (Entwicklung, Herstellung und
  Anwendung kosmetischer Mittel), edité par
  Wilfried Umbach aux editions Thieme, ISBN:
  3137126010, page 72
- Handbook of Chemistry and Physics, 73rd
  edition, page 6.10
- Harry's Cosmeticology, 7th edition, pages
  821-831
- Grundalgen und Rezpeturen der Kosmetika, par
  Karlheinz Schrader, page 238
- Grundalgen und Rezepturen der Kosmetika, par
  Karlheinz Schrader, page 238

Remarques:
Le dossier contient des informations techniques
présentées postérieurement au dépôt de la
demande et ne figurant pas dans le présent
fascicule.

**Description**

L'invention a pour objet de nouveaux dispositifs pressurisés comprenant des émulsions huile dans eau, ayant un bon pouvoir moussant, à usage topique, destinées plus particulièrement au nettoyage et au soin de la peau.

Le nettoyage de la peau est très important pour le soin du visage. Il doit être le plus performant possible car les résidus gras tels que l'excès de sébum, les restes des produits cosmétiques utilisés quotidiennement et les produits de maquillage notamment les produits «waterproof» résistants à l'eau, s'accumulent dans les replis cutanés et peuvent obstruer les pores de la peau et entraîner l'apparition de boutons.

On connaît deux grands types de produits de nettoyage de la peau : les lotions et les gels aqueux détergents moussants et les huiles et les gels anhydres nettoyants rinçables.

Les lotions et les gels aqueux détergents moussants ont une action nettoyante grâce aux tensioactifs qui mettent en suspension les résidus gras et les pigments des produits de maquillage. Ils sont efficaces et agréables cosmétiquement du fait qu'ils moussent et qu'ils sont facilement éliminés. Dans la mesure où ils ne contiennent pas d'huile cosmétique, ils présentent l'inconvénient d'assécher la peau par leur action délipidante. C'est le cas par exemple des produits décrits par le document WO95/05769, qui enseigne des lotions pour le nettoyage de la peau, très fluides, pressurisables, produisant une belle mousse, mais ces produits détruisent le film hydro-lipidique de la peau et laissent la peau propre mais rêche.

Les huiles et les gels anhydres rinçables ont une action nettoyante grâce aux huiles contenues dans ces formulations. Ces huiles permettent la solubilisation des résidus gras et la dispersion des pigments de maquillage. Ces produits sont efficaces et bien tolérés. Ils présentent l'inconvénient d'être lourds, de ne pas mousser et de ne pas conférer de sensation de fraîcheur à l'application, ce qui est pénalisant d'un point de vue cosmétique.

On a cherché à résoudre ces problèmes techniques en réalisant des crèmes et des laits démaquillants contenant à la fois des huiles, des émulsionnants et des tensio-actifs détergents en quantité suffisamment faible pour ne pas déstabiliser l'émulsion. Ces produits en dépit de leur bonne efficacité ne sont pas moussants et présentent une rinçabilité insuffisante qui nécessite l'emploi d'une lotion tonique détergente complémentaire pour parfaire le rinçage et l'élimination des salissures. Outre son caractère astreignant, l'utilisation de ce deuxième produit peut entraîner à long terme un assèchement de la peau.

On cherche à concevoir des produits détergents moussants, parfaitement rinçables à l'eau, contenant des tensio-actifs hydrophiles et des huiles permettant à la fois de rendre optimal le nettoyage de la peau et d'hydrater et de nourrir celle-ci afin d'éviter tout phénomène d'assèchement.

Pour réaliser un produit qui nettoie la peau sans l'assécher, on ne peut pas simplement introduire des huiles dans une lotion ou gel aqueux détergent. En effet, les huiles ont tendance à inhiber les propriétés moussantes de ces formulations ; on dit que les huiles «tuent» la mousse. De plus, la dispersion de l'huile est instable. On connaît, par exemple par le document WO95/17163, des émulsions moussantes, par exemple des émulsions démaquillantes. Ce type de produit est très doux, très bien toléré par la peau, mais le pouvoir moussant de ces émulsions est faible à cause de la présence des huiles. En outre, ces émulsions sont toujours opaques et relativement épaisses, ce qui ne permet pas de les conditionner dans des récipients pressurisés

Pour obtenir un bon pouvoir moussant, on ne peut pas non plus simplement introduire des tensio-actifs moussants dans les crèmes ou les laits démaquillants classiques, qui sont des émulsions huile-dans-eau. Ces tensio-actifs, introduits à des concentrations supérieures à 5% en poids, entraînent le déphasage de ces émulsions car ils perturbent le film interfacial que forme l'émulsionnant autour des gouttelettes d'huile en dispersion.

En outre, on a cherché à préparer un produit pressurisable : en effet, les conditionnements en aérosol sont particulièrement intéressants puisqu'ils permettent une distribution contrôlée, une meilleure conservation du produit, en outre, ils présentent un aspect ludique pour l'utilisateur.

On sait, par exemple par le document WO89/11907, que la méthode d'inversion de phase permet de préparer des émulsions huile dans eau, fluides, stables, ultrafines, pressurisables. Ces émulsions sont souvent conditionnées sous forme de composition pressurisée et restituées sous forme de sprays, sans solvant et sans composés organiques volatiles. Mais les émulsions préparées par la méthode d'inversion de phase dans l'art antérieur sont non moussantes. En effet, les tensioactifs employés pour faire des émulsions susceptibles de s'inverser, ou émulsions PIT, sont des non-ioniques non-moussants, essentiellement des alcools gras polyéthoxylés, les autres tensioactifs ne permettant pas d'obtenir une inversion de phase. On sait également par les nombreux travaux qui ont été développés autour de ce sujet, que le comportement des émulsions PIT, est très sensible aux composants qu'on leur incorpore, une émulsion pouvant très facilement perdre son caractère inversable et se trouver déstabilisée sous l'effet de certains additifs. A ce sujet, on pourra par exemple se reporter aux documents T. Mitsui & al «Bulletin of the chemical society of Japan», vol. 43, 3044-3048 (1970) et T.Mitsui & al «American Cosmetics and Perfumery», vol. 87, 33-36 (1972). Les émulsions PIT étaient jusqu'alors très appréciées pour leur caractère fluide qui leur confère une grande facilité d'application et d'étalement sur la peau.

On connait, par exemple par le document DE-A-4318171, des compositions aqueuses nettoyantes pour le corps et les cheveux, ces compositions comprenant de forts taux de tensio-actifs ioniques ainsi qu'une huile, qui est introduite dans la composition aqueuse tensio-active sous la forme d'une émulsion ultrafine. Ce document ne mentionne ni ne suggère la possibilité de pressuriser de telles compositions.

Aussi, c'est avec étonnement que la demanderesse a remédié aux inconvénients de l'art antérieur en mettant en oeuvre des émulsions huile dans eau (que l'on désignera H/E) spécifiques dites "ultrafines", pour lesquelles la taille des globules constituant la phase grasse est comprise dans des limites bien déterminées, lesdites émulsions ultrafines de type H/E étant elles-mêmes de préférence obtenues selon la technique dite "d'inversion de phase" qui sera détaillée par la suite. Ces émulsions H/E ultrafines sont utilisables en particulier pour le nettoyage et le soin de la peau, elles sont riches en huiles, fluides, pressurisables, moussantes, stables, translucides. Elles peuvent être pressurisées et conditionnées dans des récipients aérosols.

L'invention a pour objet un dispositif aérosol constitué d'un récipient pressurisable muni d'un moyen de diffusion comprenant une valve, ledit récipient contenant un gaz propulseur et une émulsion moussante, comprenant en poids de chacun des composants par rapport au poids total de l'émulsion :

(A) 10 à 30% d'au moins une huile cosmétique,
(B) 2 à 10% d'au moins un émulsionnant non ionique de HLB compris entre 9 et 18,
(C) 5 à 15% d'au moins un tensioactif moussant choisi parmi :

-    les alkyl phosphates,
-    les alkyl taurates,
-    les sulfosuccinates,
-    les alkyl sulfates,
-    les sarcosinates,
-    les alkyl éthersulfates,
-    les iséthionates,
-    les alkyléther carboxylates,
-    les alkyl éther de polyglycéryle,
-    les alkyl glucosides,
-    les oxydes d'amine
-    les sels d'ammonium quaternaires,
-    les bétaïnes,

(D) 40 à 60% d'eau,

la taille moyenne des globules qui constituent la phase huileuse de ces émulsions étant comprise entre 50 et 1000nm,
et la viscosité de l'émulsion mesurée à 25°C à l'aide d'un viscosimètre Brookfield équipé d'un module I tournant à une vitesse de 200 tours/minute, étant inférieure ou égale à 20 mPa.s.

Lorsque l'on exerce une pression sur le moyen de diffusion du dispositif selon l'invention, on actionne la valve et le dispositif restitue son contenu sous la forme d'une mousse onctueuse. Des produits d'une telle consistance sont particulièrement appréciés pour le nettoyage et le soin de la peau ou des cheveux.

Habituellement un tel dispositif contient 0,5 à 20% de gaz propulseur et 80 à 99,5% d'émulsion. On peut utiliser dans les dispositifs selon l'invention tous les gaz propulseurs connus pour de telles applications. On peut citer en particulier les gaz hydrocarbonés, comme par exemple le propane, l'isopropane le n-butane, l'isobutane et leurs mélanges ; les gaz fluorés comme par exemple le chlorodifluoro méthane, le dichlorodifluorométhane, le difluoroéthane, le chlorodifluoroéthane, le dichlorotétrafluoroéthane etc... et leurs mélanges ; l'azote et le dioxyde de carbone et leurs mélanges peuvent également être utilisés comme gaz propulseurs dans la présente invention.

De façon préférentielle, les émulsions selon l'invention sont conditionnées dans des récipients aérosols transparents. De tels récipients sont bien connus de l'homme du métier et d'utilisation courante. On pourra à ce sujet se référer à la demande de brevet WO95/05796. De tels récipients mettent particulièrement en valeur le caractère surprenant de l'invention : au travers du récipient aérosol on observe une émulsion translucide, bleutée, ayant une fluidité comparable à celle de l'eau, ladite émulsion étant restituée sous la forme d'une mousse onctueuse au travers du moyen de diffusion, et non sous la forme d'un spray comme on s'y attend à priori.

La viscosité Brookfield des émulsions selon l'invention est avantageusement inférieure ou égale à 20 mPa.s, et préférentiellement elle est inférieure ou égale à 10 mPa.s.

Avantageusement dans les émulsions selon l'invention, le rapport des quantités, mesurées en poids, de l'huile par rapport au tensio-actif moussant est supérieur ou égal à 0,5, préférentiellement supérieur ou égal à 1 et encore plus préférentiellement supérieur ou égal à 2.

L'invention a également pour objet de nouvelles émulsions huile dans eau, pressurisables moussantes comprenant en poids de chacun des composants par rapport au poids total de l'émulsion :

(A) 10 à 30% d'au moins une huile cosmétique,
(B) 2 à 10% d'au moins un émulsionnant non ionique de HLB compris entre 9 et 18,
(C) 5 à 15% d'au moins un tensioactif moussant choisi parmi :

-    les alkyl phosphates,
-    les alkyl taurates,
-    les sulfosuccinates,
-    les alkyl sulfates,
-    les sarcosinates,

- les alkyl éthersulfates,
- les iséthionates,
- les alkyléther carboxylates,
- les alkyl éther de polyglycéryle,
- les alkyl glucosides,
- les oxydes d'amine
- les sels d'ammonium quaternaires,
- les bétaïnes,

(D) 40 à 60% d'eau,

la taille moyenne des globules qui constituent la phase huileuse de ces émulsions étant comprise entre 50 et 1000nm,

et la viscosité de l'émulsion mesurée à 25°C à l'aide d'un viscosimètre Brookfield équipé d'un module I tournant à une vitesse de 200 tours/minute étant inférieure ou égale à 20 mPa.s.

De façon préférentielle, la taille des particules huileuses de l'émulsion est comprise entre 70 et 350nm, et encore plus préférentiellement entre 100 et 300nm. Les émulsions selon l'invention sont extrêmement stables, ont une fluidité comparable à celle de l'eau, sont translucides.

De façon habituelle, les émulsions selon l'invention sont caractérisées par le fait que leur polydispersité est très faible. En règle générale, 90% des particules des émulsions "ultrafines" selon l'invention ont une taille comprise entre 100nm et 300nm. La différence de taille entre les plus grandes et les plus petites particules est généralement comprise entre 20nm et 400nm, et préférentiellement entre 30nm et 200nm, alors que dans des émulsions classiques (autres qu'émulsions PIT ou microémulsions) la différence de taille entre les plus grandes et les plus petites particules peut atteindre des valeurs supérieures à 1000nm.

Comme indiqué précédemment, ce procédé repose sur la technique de fabrication des émulsions H/E par inversion de phase. Cette technique est, dans son principe, bien connue de l'homme de l'art et est notamment décrite dans l'article "Phase Inversion Emusification", par Th Förster et al, paru dans Cosmetics & Toiletries, vol. 106, Décembre 1991, pp 49-52. Son principe est ainsi le suivant : on prépare une émulsion (introduction de l'eau dans l'huile) à une température qui doit être supérieure à la température d'inversion de phase (ou PIT) du système, c'est à dire la température à laquelle l'équilibre entre les propriétés hydrophiles et lipophiles du ou des émulsionnants mis en oeuvre est atteint ; à température élevée (>PIT), l'émulsion est de type eau-dans-huile, et au cours de son refroidissement, à la température d'inversion de phase, cette émulsion s'inverse pour devenir une émulsion de type cette fois huile-dans-eau, et ceci en étant passée auparavant par un état de microémulsion.

Une émulsion selon l'invention peut être obtenue par un procédé d'inversion de phase caractérisé en ce que l'on mélange

(A) au moins une huile cosmétique,
(B) au moins un émulsionnant non ionique de HLB compris entre 9 et 18,
(D) de l'eau,
et éventuellement
(E) des adjuvants stables à la température d'inversion de phase,

afin d'obtenir une émulsion classique puis que l'on chauffe cette émulsion à une température située à l'intérieur ou au dessus du domaine d'inversion de phase ou que l'on prépare l'émulsion à une telle température, puis que dans un second temps on refroidit l'émulsion à une température inférieure au domaine d'inversion de phase, que l'on introduit dans cette émulsion (C) au moins un tensioactif moussant pour obtenir un mélange homogène, et qu'éventuellement on dilue encore l'émulsion obtenue avec de l'eau et on ajoute encore (E) des adjuvants.

Le domaine de température d'inversion de phase est établi pour une composition donnée en mesurant la conductibilité d'un échantillon de la composition que l'on chauffe. Lorsque l'on atteint le domaine d'inversion de phase la conductibilité de l'émulsion augmente de façon très rapide : dans le domaine d'inversion de phase on peut observer une augmentation de la conductibilité d'environ 50 micro-siemens par centimètre sur un intervalle de température de 5 à 15°C, tandis qu'elle ne sera que d'environ 5 micro-siemens par centimètre sur un intervalle de température équivalent en dehors du domaine d'inversion de phase.

La nature de la phase grasse rentrant dans la composition des émulsions selon l'invention n'est pas critique et elle peut ainsi être constituée par tous les composés qui sont déjà connus de façon générale comme convenant pour la fabrication d'émulsions de type huile dans eau. En particulier, ces composés peuvent être choisis, seuls ou en mélanges, parmi les différents corps gras, les huiles d'origine végétale, animale ou minérale, les cires naturelles ou synthétiques, et analogues.

On peut citer parmi les huiles utilisables dans la présente invention les huiles d'origine végétale ou animale, comme par exemple le perhydrosqualène, le squalane, l'huile de coprah, l'huile de macadamia, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; des huiles d'hydrocarbures telles que des huiles de paraffine, la vaseline ; des huiles de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique ; des mono et des di-esters répondant à l'une des formules (I) (II) ou (III) suivantes :

$$(I) \quad R^1\text{-}COOR^2$$

$$(II) \quad R^2OOC\text{-}R^3\text{-}COOR^2$$

$$(III) \quad R^1\text{-}COOR^3OOC\text{-}R^1,$$

dans lesquelles $R^1$ et $R^2$ représentent des groupements alkyl ayant de 1 à 22 atomes de carbone ou alcène ayant de 8 à 22 atomes de carbone, $R^3$ représente un groupement alcane di-yle ayant de 2 à 16 atomes de carbones, lesdits esters comprenant au moins 10 atomes de carbone ; parmi ces derniers composés on peut citer en particulier le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diéthylhexyle, le malate de diisostéaryle, le lactate de 2-octyl dodécyle, le triisostéarate de glycérine, le triisostéarate de glycérine, l'adipate de di-n-butyle, l'adipate de di-(2-éthyl hexyle), le dioléate d'éthylène glycol, le di-isotridécanoate d'éthylène glycol, le di-isostéarate d'éthylène glycol, le di-caprylate de néopentylglycol.

Bien entendu, la phase grasse peut également contenir un ou plusieurs adjuvants cosmétiques lipophiles classiques.

Les émulsionnants non-ioniques utilisables dans la présente invention peuvent être choisis parmi les composés constitués d'une part d'un reste lipophile choisi par exemple parmi les fonctions alkyl ou acyl en $C_6$-$C_{30}$ et d'autre part d'un reste hydrophile choisi par exemple parmi les groupements glycol, glucose et les éthers de polyols. Leur balance HLB peut aller de 9 à 18, et préférentiellement on choisit un émulsionnant de HLB allant de 9,5 à 11,5. On calcule la balance HLB (balance hydrophile-lipophile) d'un émulsionnant suivant la formule suivante :

$$HLB = \frac{100\text{-}L}{5}$$

dans laquelle L représente le pourcentage en poids du groupement lipophile (soit du groupement alkyle ou acyle en $C_6$-$C_{30}$) par rapport au poids de la molécule entière.

On peut citer en particulier comme émulsionnants non-ioniques préférentiellement utilisés dans la présente invention, les produits d'addition d'oxyde d'éthylène sur des alcools gras ayant 6 à 30 atomes de carbone ou sur des esters partiels de polyols ayant 3 à 16 atomes de carbone et d'acides gras ayant 14 à 22 atomes de carbone. Les produits d'addition d'oxyde d'éthylène sur des alcools gras sont disponibles dans le commerce. Les produits d'addition d'oxyde d'éthylène sur des esters partiels de polyols et d'acides gras peuvent être obtenus facilement par éthoxylation d'esters partiels d'acides gras du glycérol ou de mono ou de di-esters d'acides gras du sorbitol.

De façon préférentielle, on utilise un émulsionnant répondant à la formule (IV) :

$$R^4\text{-}(O\text{-}CH_2\text{-}CH_2)_n\text{-}OH \quad (IV)$$

dans laquelle $R^4$ représente un résidu hydrocarboné saturé ou insaturé, ramifié ou linéaire, ayant de 8 à 28 atomes de carbone et n représente un nombre allant de 8 à 50, préférentiellement de 8 à 30; on peut également utiliser un produit d'addition de 4 à 20 moles d'oxyde d'éthylène sur un ou plusieurs esters partiels de glycérol. Par esters partiels de glycérol on entend par exemple les mélanges de mono, di- et tri-glycérides d'acides gras en $C_{10}$-$C_{20}$ obtenus par estérification d'une mole de glycérol par 1 ou 2 moles d'un acide gras en $C_{10}$-$C_{20}$. De façon préférentielle, on utilisera comme émulsionnant le produit de condensation de l'alcool béhénylique et de 9 oxydes d'éthylène.

Les émulsions selon l'invention comprennent au moins un tensioactif moussant que l'on choisit parmi les tensioactifs moussants anioniques, cationiques, non ioniques et les amphotères.

Les tensio-actifs moussants utilisés selon la présente invention sont choisis parmi ceux possédant un pouvoir moussant caractérisé par une hauteur de mousse supérieure à 10 mm mesurée selon la méthode ROSS-MILES pour une solution à 0,02 % en poids de tensio-actif (matière active) dans de l'eau distillée à 25° C.

Parmi les tensio-actifs moussants plus particulièrement utilisables dans la présente invention, on peut citer :
Dans la catégorie des anioniques moussants :

- les alkyl phosphates, comme par exemple le lauryl phosphate de sodium,
- les alkyl taurates, comme par exemple le méthyl palmitoyl taurate de sodium,
- les sulfosuccinates, comme par exemple le sulfosuccinate de cocoyl, le sel disodique du sulfosuccinate d'alcool laurique oxyéthyléné,
- les alkyl sulfates, comme par exemple le lauryl sulfate de triéthanolamine,
- les sarcosinates, comme par exemple le lauroyl sarcosinate de sodium,
- les alkyl éthersulfates, comme par exemple le lauroyl éther sulfate de sodium,

- les iséthionates, comme par exemple le cocoyl iséthionate de sodium,
- les alkyléther carboxylates, comme par exemple le décyl éther carboxylate de sodium oxyéthyléné.

Dans la catégorie des non-ioniques moussants :

- les alkyl éther de polyglycéryle, comme par exemple le dodécane diol polyglycérolé,
- les alkyl glucosides, comme par exemple le décyl glucoside.

Dans la catégorie des cationiques moussants :

- les oxydes d'amine
- les sels d'ammonium quaternaires, comme par exemple le polyquaternium 22 (nomenclature INCI),

Dans la catégorie des amphotères moussants :

- les bétaïnes, comme par exemple le di-sodium cocoamphodiacétate, le cocamidopropylbétaïne, le cocobétaïne.

En outre les émulsions selon l'invention peuvent comprendre au moins un co-émulsionnant en quantité telle que l'ensemble constitué des émulsionnants et coémulsionnants représente de 0,5 à 30% du total de la composition, et préférentiellement de 2 à 10% de la composition. Les coémulsionnants peuvent représenter jusqu'à 50% en poids de l'ensemble constitué des émulsionnants et coémulsionnants. Ce co-émulsionnant est choisi parmi les alcools gras en $C_{12}$-$C_{22}$ ou les esters partiels de polyols en $C_2$-$C_6$ avec des acides gras en $C_{12}$-$C_{22}$. Préférentiellement on choisit d'utiliser des esters gras de glycérol en $C_{12}$-$C_{22}$.

Les émulsions selon l'invention comprennent de 10 à 90% d'eau, et préférentiellement de 40 à 60%. Habituellement on entend par eau de l'eau pure. Toutefois, une partie de l'eau utilisée dans les émulsions selon l'invention peut éventuellement être choisie parmi les eaux minérales ou thermales. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'elles contiennent, tels que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oligo-élémentaires préparées à partir d'eau purifiée (déminéralisée ou distillée).

Une eau thermale ou minérale naturelle utilisée selon l'invention peut, par exemple être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains.

De manière classique, la phase aqueuse dispersante peut être constituée par de l'eau, ou un mélange d'eau et d'alcool(s) polyhydrique(s) comme par exemple glycérol, propylène glycol et sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvants cosmétiques classiques hydrosolubles. Cette phase aqueuse représente habituellement de 50 à 95% de la composition, et préférentiellement de 70 à 90%.

Les émulsions cosmétiques ou dermatologiques de l'invention peuvent, en outre, contenir

(E) des adjuvants hydrosolubles ou liposolubles habituels dans le domaine cosmétique tels que les conservateurs, les antioxydants, les parfums, les filtres, les matières colorantes, les actifs hydrophiles ou lipophiles.

Les actifs pour la peau peuvent être des actifs anti-âge, des actifs anti-rides, des hydratants ou des humectants, des actifs amincissants, des actifs dépigmentants, des actifs anti-radicaux libres (espèces radicalaires de l'oxygène), des actifs nutritifs, des actifs protecteurs, des actifs restructurants, des actifs raffermissants, des actifs antiacnéiques, des actifs exfoliants, des actifs émollients ou encore des actifs traitants des maladies de peau comme les mycoses, les dermites, le psoriasis, etc. Ces actifs sont utilisés, selon leur nature, dans les proportions habituelles des microémulsions, et par exemple de 0,01% à 10% en poids par rapport au poids total de la microémulsion.

Un quatrième objet de la présente invention est constitué par l'utilisation des émulsions selon l'invention telles que ci-dessus définies comme, ou pour la fabrication de, compositions cosmétiques et/ou dermatologiques pour application sur la peau ou les cheveux, en particulier pour le nettoyage et le soin de la peau.

Le procédé de traitement cosmétique de la peau ou des cheveux consiste à appliquer sur ceux-ci une quantité efficace d'une composition cosmétique telle que définie ci-dessus.

Des exemples sont donnés ci-dessous afin d'illustrer l'objet de l'invention.

## EXEMPLES

Les pourcentages donnés dans les exemples sont calculés en poids.

La viscosité est donnée en mPa.s. Elle est mesurée à 25°C à l'aide d'un viscosimètre Brookfield équipé d'un module (module I) tournant à une vitesse de 200tours/minute.

**Exemple 1 :**

*Phase 1*

- éther dicaprylique     7,7%
- stéarate d'isocétyle     3%
- isononanoate de cetéaryle     4%
- beheneth-9     4,5%

*Phase 2*

- eau distillée     14,5%
- conservateur     qs

*Phase 3*

- eau distillée     qsp100
- lauryl éther sulfate de sodium     5%

**Mode opératoire** : On chauffe séparément à 60°C et on homogénéise les phases 1 et 2. Sous agitation lente on verse lentement la phase 2 sur la phase 1 et on chauffe le mélange jusqu'à la température d'inversion de phase qui est autour de 85°C. L'émulsion obtenue devient quasi-transparente et bleutée. On arrête le chauffage et on verse la phase 3 non chauffée et on laisse refroidir le mélange en gardant une agitation lente. La viscosité de l'émulsion est de 3,5 mPa.s.

La composition obtenue est introduite dans un récipient aérosol que l'on pressurise avec de l'isobutane en proportion en poids 97/3. L'ensemble est translucide et parfaitement stable après plusieurs mois de stockage. Une pression exercée sur la tête de distribution du récipient permet de restituer une mousse onctueuse que l'on utilise comme nettoyant hydratant pour le visage.

**Exemple 2 :**

*Phase 1*

- cétéareth-12     3%
- stéarate de glycéryle     2%
- palmitate d'octyle     10%
- adipate de di-octyle     10%

*Phase 2*

- eau distillée     20%
- glycérol     3%

*Phase 3*

- eau distillée     qsp100
- conservateur     qs
- décyl polyglucose     5%
- cocoyl amidopropyl bétaïne     5%

On procède suivant le même mode opératoire qu'à l'exemple 1, pour obtenir une émulsion moussante démaquillante. La viscosité de l'émulsion est de 5,9 mPa.s. Elle est restituée sous forme d'une mousse onctueuse qui laisse la peau propre et douce après application et rinçage.

**Exemple 3 :**

*Phase 1*

- éther dicaprylique     7%
- stéarate d'isocétyle     7%
- laurate d'hexyle     1,5%
- beheneth-9     4,5%

*Phase 2*

- eau distillée     17,5%
- pyrrolidone carboxylate de sodium     3

*Phase 3*

- eau distillée     qsp100
- conservateur     qs
- lauryl éther sulfate de sodium     5%
- décyl glucoside     3%

On procède suivant le même mode opératoire qu'à l'exemple 1, pour obtenir une émulsion moussante hydratante. La viscosité de l'émulsion est de 2,6 mPa.s. Elle est restituée sous forme d'une mousse onctueuse.

**Revendications**

1.  Dispositif aérosol constitué d'un récipient pressurisable muni d'un moyen de diffusion, caractérisé en ce que ce récipient contient un gaz propulseur et une émulsion huile dans eau, moussante, comprenant en poids de chacun des composants par rapport au poids total de l'émulsion :

    (A) 10 à 30% d'au moins une huile cosmétique,
    (B) 2 à 10% d'au moins un émulsionnant non ionique de HLB compris entre 9 et 18,

(C) 5 à 15% d'au moins un tensioactif moussant choisi parmi :

- les alkyl phosphates,
- les alkyl taurates,
- les sulfosuccinates,
- les alkyl sulfates,
- les sarcosinates,
- les alkyl éthersulfates,
- les iséthionates,
- les alkyléther carboxylates,
- les alkyl éther de polyglycéryle,
- les alkyl glucosides,
- les oxydes d'amine
- les sels d'ammonium quaternaires,
- les bétaïnes,

(D) 40 à 60% d'eau,

la taille moyenne des globules qui constituent la phase huileuse de ladite émulsion étant comprise entre 50 et 1000nm,
et la viscosité de l'émulsion mesurée à 25°C à l'aide d'un viscosimètre Brookfield équipé d'un module I tournant à une vitesse de 200tours/minute, étant inférieure ou égale à 20 mPa.s.

2. Dispositif selon la revendication 1, caractérisé en ce que l'émulsion a été préparée par la méthode d'inversion de phase.

3. Dispositif selon l'une quelconque des revendications précédentes, caractérisé en ce que la taille des particules huileuses de l'émulsion est comprise entre 70 et 350nm.

4. Dispositif selon l'une quelconque des revendications précédentes, caractérisé en ce que la taille des particules huileuses est comprise entre 100 et 300nm.

5. Dispositif selon l'une quelconque des revendications précédentes, caractérisé en ce que 90% des particules ont une taille comprise entre 100 et 300nm.

6. Dispositif selon l'une quelconque des revendications précédentes, caractérisé en ce que la viscosité de l'émulsion est inférieure ou égale à 10 mPa.s.

7. Dispositif selon l'une quelconque des revendications précédentes, caractérisé en ce que le rapport des quantités, mesurées en poids, de l'huile par rapport au tensio-actif moussant est supérieur ou égal à 0,5, préférentiellement supérieur ou égal à 1 et encore plus préférentiellement supérieur ou égal à 2.

8. Dispositif selon l'une quelconque des revendications précédentes, caractérisé en ce que l'émulsionnant non-ionique est choisi parmi les composés constitués d'une part d'un reste lipophile choisi parmi les fonctions alkyl ou acyl en $C_6$-$C_{30}$ et d'autre part d'un reste hydrophile choisi parmi les groupements glycol, glucose et les éthers de polyols.

9. Dispositif selon l'une quelconque des revendications précédentes, caractérisé en ce que l'émulsionnant est choisi parmi les non-ioniques de balance HLB allant de 9,5 à 11,5.

10. Dispositif selon l'une quelconque des revendications précédentes, caractérisé en ce que l'émulsionnant est choisi parmi les produits répondant à la formule (IV) :

$$R^4\text{-(O-CH}_2\text{-CH}_2)_n\text{-OH} \qquad \text{(IV)}$$

dans laquelle $R^4$ représente un résidu hydrocarboné saturé ou insaturé, ramifié ou linéaire, ayant de 8 à 28 atomes de carbone et n représente un nombre allant de 8 à 50 et les produits d'addition de 4 à 20 moles d'oxyde d'éthylène sur un ou plusieurs esters partiels de glycérol.

11. Dispositif selon l'une quelconque des revendications précédentes, caractérisé en ce que le tensioactif moussant est choisi parmi les tensio-actifs moussants possédant un pouvoir moussant caractérisé par une hauteur de mousse supérieure à 10 mm mesurée selon la méthode ROSS-MILES pour une solution à 0,02 % en poids de tensio-actif dans de l'eau distillée à 25° C.

12. Dispositif selon l'une quelconque des revendications précédentes, caractérisé en ce qu'elle comprend jusqu'à une partie en poids par rapport au poids d'émulsionnant d'au moins un co-émulsionnant choisi parmi les alcools gras en $C_{12}$-$C_{22}$ et les esters partiels de polyols en $C_2$-$C_6$ avec des acides gras en $C_{12}$-$C_{22}$.

13. Dispositif selon la revendication précédente, caractérisé en ce que le co-émulsionnant est présent en quantité telle que l'ensemble constitué des émulsionnants et co-émulsionnants représente de 0,5 à 30% du total de la composition, et préférentiellement de 2 à 10%.

14. Dispositif selon l'une quelconque des revendications précédentes, caractérisé en ce que l'émulsion comprend une eau minérale ou thermale.

**15.** Dispositif selon l'une quelconque des revendications précédentes, caractérisé en ce que l'émulsion comprend en outre des adjuvants hydrosolubles ou liposolubles habituels dans le domaine cosmétique tels que les conservateurs, les antioxydants, les parfums, les filtres, les matières colorantes, les actifs hydrophiles ou lipophiles.

**16.** Dispositif selon l'une quelconque des revendications précédentes, caractérisé en ce que le récipient aérosol est transparent.

**17.** Emulsion huile dans eau, pressurisable moussante comprenant, en poids de chacun des composants par rapport au poids total de l'émulsion :

(A) 10 à 30% d'au moins une huile cosmétique,
(B) 2 à 10% d'au moins un émulsionnant non ionique de HLB compris entre 9 et 18,
(C) 5 à 15% d'au moins un tensioactif moussant choisi parmi :

- les alkyl phosphates,
- les alkyl taurates,
- les sulfosuccinates,
- les alkyl sulfates,
- les sarcosinates,
- les alkyl éthersulfates,
- les iséthionates,
- les alkyléther carboxylates,
- les alkyl éther de polyglycéryle,
- les alkyl glucosides,
- les oxydes d'amine
- les sels d'ammonium quaternaires,
- les bétaïnes,

(D) 40 à 60% d'eau,

la taille moyenne des globules qui constituent la phase huileuse de ces émulsions étant comprise entre 50 et 1000nm,
et la viscosité de l'émulsion mesurée à 25°C à l'aide d'un viscosimètre Brookfield équipé d'un module I tournant à une vitesse de 200 tours/minute, étant inférieure ou égale à 20 mPa.s.

**18.** Utilisation d'une émulsion selon la revendication 17 pour la fabrication de compositions cosmétiques et/ou dermatologiques destinées à être appliquées sur la peau ou les cheveux, et en particulier destinées au nettoyage et/ou au soin de la peau.

**Claims**

**1.** Aerosol device composed of a pressurizable container equipped with a dispensing means, characterized in that this container comprises a propellant gas and a foaming oil-in-water emulsion comprising, by weight of each of the components with respect to the total weight of the emulsion:

(A) 10 to 30% of at least one cosmetic oil,
(B) 2 to 10% of at least one nonionic emulsifier with an HLB of between 9 and 18,
(C) 5 to 15% of at least one foaming surfactant chosen from:

- alkyl phosphates,
- alkyltaurates,
- sulphosuccinates,
- alkyl sulphates,
- sarcosinates,
- alkyl ether sulphates,
- isethionates,
- alkyl ether carboxylates,
- polyglyceryl alkyl ethers,
- alkyl glucosides,
- amine oxides,
- quaternary ammonium salts,
- betaines,

(D) 40 to 60% of water,

the mean size of the globules which constitute the oily phase of the said emulsion being between 50 and 1000 nm,
and the viscosity of the emulsion, measured at 25°C using a Brookfield viscometer equipped with a rotor I rotating at a speed of 200 revolutions/minute, being less than or equal to 20 mPa·s.

**2.** Device according to Claim 1, characterized in that the emulsion was prepared by the phase inversion method.

**3.** Device according to either one of the preceding claims, characterized in that the size of the oily particles of the emulsion is between 70 and 350 nm.

**4.** Device according to any one of the preceding claims, characterized in that the size of the oily particles is between 100 and 300 nm.

**5.** Device according to any one of the preceding claims, characterized in that 90% of the particles have a size of between 100 and 300 nm.

**6.** Device according to any one of the preceding claims, characterized in that the viscosity of the emulsion is less than or equal to 10 mPa·s.

**7.** Device according to any one of the preceding claims, characterized in that the ratio of the amounts, measured by weight, of the oil with respect to the foaming surfactant is greater than or equal to 0.5, preferably greater than or equal to 1 and more preferably still greater than or equal to 2.

8. Device according to any one of the preceding claims, characterized in that the nonionic emulsifier is chosen from compounds composed, on the one hand, of a lipophilic residue chosen from $C_6$-$C_{30}$ alkyl or acyl functional groups and, on the other hand, of a hydrophilic residue chosen from glycol or glucose groups and polyol ethers.

9. Device according to any one of the preceding claims, characterized in that the emulsifier is chosen from nonionic emulsifiers with an HLB ranging from 9.5 to 11.5.

10. Device according to any one of the preceding claims, characterized in that the emulsifier is chosen from products corresponding to the formula (IV):

$$R^4\text{-}(O\text{-}CH_2\text{-}CH_2)_n\text{-}OH \qquad (IV)$$

in which $R^4$ represents a saturated or unsaturated and branched or unbranched hydrocarbonaceous residue having from 8 to 28 carbon atoms and n represents a number ranging from 8 to 50, and addition products of 4 to 20 mol of ethylene oxide with one or more glycerol partial esters.

11. Device according to any one of the preceding claims, characterized in that the foaming surfactant is chosen from foaming surfactants which have a foaming power characterized by a foam height of greater than 10 mm, measured according to the Ross-Miles method for a 0.02% by weight solution of surfactant in distilled water at 25°C.

12. Device according to any one of the preceding claims, characterized in that it comprises up to one part by weight with respect to the weight of emulsifier of at least one coemulsifier chosen from $C_{12}$-$C_{22}$ fatty alcohols and partial esters of $C_2$-$C_6$ polyols with $C_{12}$-$C_{22}$ fatty acids.

13. Device according to the preceding claim, characterized in that the coemulsifier is present in an amount such that the combination composed of the emulsifiers and coemulsifiers represents from 0.5 to 30% of the total of the composition and preferably from 2 to 10%.

14. Device according to any one of the preceding claims, characterized in that the emulsion comprises a mineral or thermal water.

15. Device according to any one of the preceding claims, characterized in that the emulsion additionally comprises water-soluble or fat-soluble adjuvants which are usual in the cosmetics field, such as preservatives, antioxidants, fragrances, screening agents, colorants, or hydrophilic or lipophilic active principles.

16. Device according to any one of the preceding claims, characterized in that the aerosol container is transparent.

17. Foaming pressurizable oil-in-water emulsion comprising, by weight of each of the components with respect to the total weight of the emulsion:

(A) 10 to 30% of at least one cosmetic oil,
(B) 2 to 10% of at least one nonionic emulsifier with an HLB of between 9 and 18,
(C) 5 to 15% of at least one foaming surfactant chosen from:

- alkyl phosphates,
- alkyltaurates,
- sulphosuccinates,
- alkyl sulphates,
- sarcosinates,
- alkyl ether sulphates,
- isethionates,
- alkyl ether carboxylates,
- polyglyceryl alkyl ethers,
- alkyl glucosides,
- amine oxides,
- quaternary ammonium salts,
- betaines,

(D) 40 to 60% of water,

the mean size of the globules which constitute the oily phase of these emulsions being between 50 and 1000 nm,
and the viscosity of the emulsion, measured at 25°C using a Brookfield viscometer equipped with a rotor I rotating at a speed of 200 revolutions/minute, being less than or equal to 20 mPa·s.

18. Use of an emulsion according to Claim 17 in the manufacture of cosmetic and/or dermatological compositions intended to be applied to the skin or hair and in particular intended for cleansing and/or caring for the skin.

**Patentansprüche**

1. Aerosolvorrichtung, die aus einem Behälter besteht, der unter Druck gesetzt werden kann und mit einer Einrichtung zum Zerstäuben versehen ist, dadurch gekennzeichnet, daß der Behälter ein Treibgas und eine schäumende Öl-in-Wasser-Emulsion enthält, die in Gewichtsprozent jeder Komponente, bezogen auf das Gesamtgewicht der Emulsion, enthält:

(A) 10 bis 30 % mindestens eines kosmetischen Öls,
(B) 2 bis 10 % mindestens eines nichtionischen Emulgators mit einem HLB-Wert von 9 bis 18,
(C) 5 bis 15 % mindestens eines schäumenden grenzflächenaktiven Stoffes, der ausgewählt ist unter:

- den Alkylphosphaten;
- den Alkyltauraten;
- den Sulfosuccinaten;
- den Alkylsulfaten;
- den Sarcosinaten;
- den Alkylethersulfaten;
- den Isethionaten;
- den Alkylethercarboxylaten;
- den Polyglycerylalkylethern;
- den Alkylglucosiden;
- den Aminoxiden;
- den quartären Ammoniumsalzen;
- den Betainen; und

(D) 40 bis 60 % Wasser,

wobei die mittlere Größe der Kügelchen, die die Ölphase der Emulsion bilden, im Bereich von 50 bis 1000 nm liegt und die mit einem Brookfield-Viskosimeter, das mit einem Modul I ausgestattet ist, das sich mit einer Geschwindigkeit von 200 U/min dreht, bestimmte Viskosität höchstens 20 mPa·s beträgt.

2. Vorrichtung nach Anspruch 1, dadurch gekennzeichnet, daß die Emulsion durch Phaseninversion hergestellt wird.

3. Vorrichtung nach einem der vorhergehenden Ansprüche, dadurch gekennzeichnet, daß die Größe der Ölpartikel der Emulsion im Bereich von 70 bis 350 nm liegt.

4. Vorrichtung nach einem der vorhergehenden Ansprüche, dadurch gekennzeichnet, daß die Größe der Ölpartikel im Bereich von 100 bis 300 nm liegt.

5. Vorrichtung nach einem der vorhergehenden Ansprüche, dadurch gekennzeichnet, daß 90 % der Partikel eine Größe von 100 bis 300 nm aufweisen.

6. Vorrichtung nach einem der vorhergehenden Ansprüche, dadurch gekennzeichnet, daß die Viskosität der Emulsion höchstens 10 mPa·s beträgt.

7. Vorrichtung nach einem der vorhergehenden Ansprüche, dadurch gekennzeichnet, daß das auf das Gewicht bezogene Verhältnis von Öl zu schäumendem grenzflächenaktiven Stoff mindestens 0,5 und vorzugsweise mindestens 1 und noch bevorzugter mindestens 2 beträgt.

8. Vorrichtung nach einem der vorhergehenden Ansprüche, dadurch gekennzeichnet, daß der nichtionische Emulgator unter den Verbindungen ausgewählt ist. die einerseits aus einer lipophilen Gruppe, die unter den $C_{6-30}$-Alkylgruppen oder $C_{6-30}$-Acylgruppen ausgewählt ist, und andererseits einer hydrophilen Gruppe aufgebaut sind, die unter Glykol, Glucose und Ethern von Polyolen ausgewählt ist.

9. Vorrichtung nach einem der vorhergehenden Ansprüche, dadurch gekennzeichnet, daß der Emulgator unter den nichtionischen Emulgatoren mit einem HLB-Wert von 9,5 bis 11,5 ausgewählt ist.

10. Vorrichtung nach einem der vorhergehenden Ansprüche, dadurch gekennzeichnet, daß der nichtionische Emulgator unter den Verbindungen, die der folgenden Formel (IV) entsprechen:

$$R^4\text{-}(O\text{-}CH_2\text{-}CH_2)_n\text{-}OH \qquad (IV),$$

worin $R^4$ eine gesättigte oder ungesättigte, verzweigte oder geradkettige Kohlenwasserstoffgruppe mit 8 bis 28 Kohlenstoffatomen und n eine Zahl von 8 bis 50 bedeutet, und den Additionsprodukten von 4 bis 20 mol Ethylenoxid mit einem oder mehreren partiellen Glycerinestern ausgewählt ist.

11. Vorrichtung nach einem der vorhergehenden Ansprüche, dadurch gekennzeichnet, daß der schäumende grenzflächenaktive Stoff unter den schäumenden grenzflächenaktives Stoffen ausgewählt, die eine für eine Lösung mit 0,02 Gew.-% des grenzflächenaktiven Stoffes in destilliertem Wasser bei 25 °C nach der ROSS-MILES-Methode bestimmte Schaumhöhe über 10 mm aufweisen.

12. Vorrichtung nach einem der vorhergehenden Ansprüche, dadurch gekennzeichnet, daß sie einige Gewichtsteile mindestens eines Coemulgators, bezogen auf das Gewicht des Emulgators enthält, der unter den $C_{12-22}$-Fettalkoholen oder den partiellen Estern von $C_{2-6}$-Polyolen und $C_{12-22}$-Fettsäuren ausgewählt ist.

13. Vorrichtung nach einem der vorhergehenden Ansprüche, dadurch gekennzeichnet, daß der Coemulgator in einer solchen Menge vorliegt, daß die Emulgatoren und Coemulgatoren zusammen 0,5 bis 30 % und vorzugsweise 2 bis 10 % des Gesamtgewichts der Zusammensetzung ausmachen.

14. Vorrichtung nach einem der vorhergehenden Ansprüche, dadurch gekennzeichnet, daß die Emulsion ein Mineralwasser oder Thermalwasser enthält.

15. Vorrichtung nach einem der vorhergehenden Ansprüche, dadurch gekennzeichnet, daß die Emulsion ferner wasserlösliche oder fettlösliche Zusatzstoffe enthält, die üblicherweise in der Kosmetik eingesetzt werden, wie Konservierungsmittel, Antioxidantien, Parfums, Filter, Färbemittel und hydrophile und lipophile Wirkstoffe.

16. Vorrichtung nach einem der vorhergehenden Ansprüche, dadurch gekennzeichnet, daß der Aerosolbehälter transparent ist.

17. Schäumende Öl-in-Wasser-Emulsion, die unter Druck gesetzt werden kann, dadurch gekennzeichnet, daß sie in Gewichtsprozent jeder Komponente, bezogen auf das Gesamtgewicht der Emulsion, enthält:

(A) 10 bis 30 % mindestens eines kosmetischen Öls,
(B) 2 bis 10 % mindestens eines nichtionischen Emulgators mit einem HLB-Wert von 9 bis 18,
5 bis 15 % mindestens eines schäumenden grenzflächenaktiven Stoffes, der ausgewählt ist unter:

- den Alkylphosphaten;
- den Alkyltauraten;
- den Sulfosuccinaten;
- den Alkylsulfaten;
- den Sarcosinaten;
- den Alkylethersulfaten;
- den Isethionaten;
- den Alkylethercarboxylaten;
- den Polyglycerylalkylethern;
- den Alkylglucosiden;
- den Aminoxiden;
- den quartären Ammoniumsalzen;
- den Betainen, und

(D) 40 bis 60 % Wasser,

wobei die mittlere Größe der Kügelchen, die die Ölphase der Emulsion bilden, im Bereich von 50 bis 1000 nm liegt und die mit einem Brookfield-Viskosimeter, das mit einem Modul I ausgestattet ist, das sich mit einer Geschwindigkeit von 200 U/min dreht, bestimmte Viskosität höchstens 20 mPa·s beträgt.

18. Verwendung einer Emulsion nach Anspruch 17 zur Herstellung von kosmetischen und/oder dermatologischen Zusammensetzungen, die dazu vorgesehen sind, auf die Haut oder die Haare aufgetragen zu werden und insbesondere zur Reinigung und/oder Pflege der Haut dienen sollen.